Europäisches Patentamt

European Patent Office

Office européen des brevets

(19)

(11) Publication number: **0 295 212**
**A2**

# (12) EUROPEAN PATENT APPLICATION

(21) Application number: 88810368.6

(22) Date of filing: 06.06.88

(51) Int. Cl.4: **A 61 K 47/00**
**A 61 K 9/42**

(30) Priority: 10.06.87 US 61057

(43) Date of publication of application:
14.12.88 Bulletin 88/50

(84) Designated Contracting States:
BE DE ES FR GB GR

(71) Applicant: **WARNER-LAMBERT COMPANY**
**201 Tabor Road**
**Morris Plains New Jersey 07950 (US)**

(72) Inventor: **Schobel, Alexander Mark**
**362A Hillcrest Road**
**Whitehouse Station New Jersey 08889 (US)**

(74) Representative: Silbiger, Jakob, Dr.
c/o CAPSUGEL AG Münchensteinerstrasse 41 Postfach
CH-4002 Basel (CH)

(54) Process for preparing a pharmaceutical composition.

(57) Process of preparing a pharmaceutical composition which comprises granulating under heat a blend containing a powdered wax matrix, leachable filler and active pharmaceutical composition until a free-flowing mixture is obtained.

EP 0 295 212 A2

## Description

### PROCESS FOR PREPARING A PHARMACEUTICAL COMPOSITION

This invention relates to a process for preparing a pharmaceutical composition of matter and more particularly to the preparation and use of a leachable non-toxic wax matrix containing pharmaceutically acceptable salts of phenindamine.

Phenindamine (2,3,4,9-Tetrahydro-2-methyl-9-phenyl-1H-indeno[2,1-c]pyridine) is a stable, white crystalline powder described in 1949 in United States Patent 2,470,108. The tartrate salt is soluble up to 3% in water, and sparingly soluble in propylene glycol. Unlike other antihistamines in common use, phenindamine does not produce drowsiness and sleepiness and on the contrary has a mild stimulating action on some patients and may even cause insomnia when taken before bedtime. The use of phenindamine, however, has been curtailed because of its inadequate antihistamine properties. This may have been due to its isomerization to an inactive form, isophenindamine. The isomerization reaction has been found to occur when phenindamine is stored in both wet and dry states and is enhanced in solutions having an alkaline pH or when formulated with oxidizing substances. Commercial formulations containing phenindamine include Nolahist and Nolamine (both trademarks of Carnrick Laboratories).

Nolahist is a film coated tablet containing 25 mg of phenindamine tartrate formulated for temporary relief of running nose, sneezing and watery eyes whereas Nolamine is a film coated tablet containing 24 mg phenindamine tartrate, 4 mg chlorpheniramine maleate, and 50 mg phenylpropanolamine hydrochloride formulated to provide 8 to 12 hours of continuous relief.

A need exists for a nonsedating, and yet mild, anti-histamine product which is storage stable for long periods of time even when phenindamine is used in the presence of alkaline buffers and known oxidizing agents.

Various attempts have been made to entrap pharmaceutically active ingredients to enable the preparation of sustained release formulations and formulations that dissolve or erode in the gastrointestinal tract.

U.S. Patent 3,402,240 describes a tablet which is useful in administrating many therapeutic agents and drugs some of which, for illustrative purposes, are organic nitrites, sympathomimetic amines, barbituric acid derivatives, salicylates, xanthine derivatives, and many others. The tablet comprises a matrix substantially insoluble in gastric and intestinal juices, a therapeutically bland or inert filler or extender and an active therapeutic agent or drug. The matrix is constituted of such materials as carnauba wax, candelilla wax, esparto wax, or ouricury wax, which have melting points between about 68° and 90°C.

The filler or extender may be any one of the standard materials used in making medicinal tablets, such as calcium or sodium phosphate dibasic, magnesium stearate (serving also as a lubricant in tableting), calcium phosphate tribasic, talc, calcium oxide, calcium stearate, sodium stearate and starch and mixtures thereof as now used in the making of tablets and methylcellulose (serving also as a swelling agent in the gastrointestinal tract).

U.S. Patent 3,402,240 discloses the preparation of tablets containing a granulation of the active component by mixing powdered carnauba wax, calcium phosphate tribasic and talc which mixture is then blended with a glucose solution to form a granulation which is partially dried and passed through a #6 sieve and again oven dried. Additional dry components and active drug are then mixed with the granulation and then pressed into tablets. It appears that this reference uses carnauba wax as a hydrophobic material to enhance limited release of water soluble materials.

U.S. Patent 2,875,130 discloses a method of preparing a sustained release pharmaceutical powder which comprises reducing a solid medicament to a particle size of a maximum of about 10 microns, mixing the thus formed particles in from about 5% to about 35% by weight of a liquefied lipid material which is substantially water insoluble and has a melting point of above about 85° C., solidifying the thus formed mixture and then reducing the solidified mixture to form a primary powder having a maximum particle size in the range of from about 5 to 25 microns. The thus formed powder is mixed with a melt of from about 25% to about 85% by weight of a lipid material which is substantially water insoluble and has a melting point which is a minimum of about 5° C. lower than the melting point of the first mentioned lipid material while maintaining the temperature of the melt below the melting point of the first mentioned lipid material and above the melting point of the second mentioned lipid material, mixing the powder-lipid mixture with water to form an emulsion while maintaining the water- powder-lipid mixture at a temperature above the melting point of the second mentioned lipid material and below the melting point of the first mentioned lipid material, cooling the emulsion to a temperature below the melting point of the second mentioned lipid material to precipitate the sustained release pharmaceutical powder, said solid medicament having a melting point higher than the second mentioned lipid material.

U.S. Patent 4,552,899 discloses pharmaceutical compositions and methods of using same comprising a non-steroidal anti-inflammatory drug in combination with at least one other active component selected from an antihistamine, decongestant, cough suppressant (antitussive) or expectorant which are provided for the relief of cough, cold and cold-like symptoms.

Copending US Application 852,471 filed April 15, 1986 overcomes the deficiencies of the prior art by preparing a pharmaceutical composition of matter for use in the treatment of sinus, allergy, and cold symptoms, which comprises an effective amount of a pharmaceutically acceptable salt of phenindamine entrapped in a leachable non-toxic wax matrix in combination with at least 1 material selected from the group consisting of analgesics, decongestants, antitussives and mixtures thereof.

The wax matrix is prepared by melting the wax and while mixing blending into the melted wax the leachable component and the phenindamine salt. The filler and drug may be added separately or simultaneously and may be added in portions or all at once. Once a homogenous mixture is obtained the mass is cooled, granulated and milled to produce a free flowing particulate material having an average particle size of about 125 to 425 microns (U.S. standard sieve size). While this composition of matter is an effective means for stabilizing the phenindamine, the process for preparing the product has distinct disadvantages, namely once the wax is melted and blended with the remaining components it is permitted to solidify upon cooling. During the cooling procedure heavy binding of the mass occurs resulting in the formation of large lumps, i.e. boulders, which are difficult to process further. In fact, a screen sieve analysis of the product reveals that 100% of the product is retained on a 10 mesh screen (US standard sieve size). This product once formed is not free flowing and is difficult to remove from the blender and process further by granulation. Even when granulated, it does not consistently produce a granular product having a uniform particle size. This invention provides for the virtual immediate release of the active pharmaceutical compound so that within 1 hour over 70% of the drug is dissolved out of the wax matrix. In addition, it was found that by using the leachable non-toxic wax matrix virtually no oxidation or isomerization of the active component occurs even in the presence of known isomerization agents, such as some of the analgesics and decongestants used in these ions.

Unlike the prior procedure, the process of the present invention involves blending the active pharmaceutical composition with a powdered non-toxic wax and a leachable filler. Blending may be done in any convenient form such that a homogenous, uniform mixture containing the three components is obtained. While not being critical, blending times of 1 minute to 1 hour have been found suitable.

Once blending is completed the mixture is heated to a temperature below the melting point of the wax. This step is critical to the invention, since heating the wax to a liquified state completely negates the advantages achieved by the invention. Suitable temperatures may range from 5° to 25°C below the melting point at which the wax will melt when it is in the form of a blend with the remaining components. The particular temperature may not also be the same as the melting point of the pure wax absent such other components. Heating is done gradually to soften the wax which is preferably done while the mixture is being mixed. Once the wax has softened, the active pharmaceutical composition and leachable filler become impregnated in the then formed wax matrix. Heating is performed gradually to prevent liquification of the system by wax melt down. As the heated mixture is blended, a free-flowing granulation is produced which has an average particle size profile that may be easily cooled and granulated into uniform particles. This material generally exhibits about 28% particles being retained on a 20 mesh sieve, about 17% being retained on a 40 mesh sieve, about 9% being retained on a 60 mesh sieve and about 2.4% being retained on 80 mesh sieve, all sieve sizes being US standard sieve sizes.

The heating and mixing steps are performed for a sufficient time to achieve the desired end result, and preferably are carried out from 3 minutes to 1 hour. Once the free-flowing granulation is prepared it is discharged and permitted to cool. Once the product is cooled to around 35°C or lower, namely room temperature (25°C) the product may be milled to the desired particle size and stored for future use or simply stored and milled, if necessary at some later time.

The compositions of this invention employ an active pharmaceutical compound entrapped within a leachable non-toxic wax matrix. The matrix is substantially insoluble in gastric and intestinal juices yet rapidly releases the drug in the presence of the same. The matrix contains a non-toxic wax, the active drug, and an inert filler or extender as the leachable component of the matrix. When the matrix passes into the intestinal tract the surface of the matrix is attacked and the filler becomes leached out of or swells within the matrix particles rendering the drug available for therapeutic action. As the gastrointestinal juices continue to attack the filler and leach out the active component, the matrix begins to disintegrate. The leaching process should occur quite rapidly, preferably within one hour to result in the release of at least 70% of the drug. In order to provide for this rapid release, the amount of matrix, leachable component and drug present must be carefully controlled.

The active pharmaceutical composition may be any drug that is affected by the presence of water and/or oxidizing agents. As indicated above phenindamine is isomerized to the inactive form, isophenindamine, in the presence of air in both wet and dry states and is enhanced in solutions having an alkaline pH or when formulated with oxidizing substances. Similarly, procaterol, (8-hydroxy-5-[1-hydroxy-2-[(1-methylethyl)amin-]butyl]-2(1H)-quinolinone) is also known to be extremely unstable when formulated with oxidizing substances or when left exposed to the atmosphere in both wet and dry states. These two active compounds along with other known drugs having similar disadvantages are considered to be within the scope of the present invention even though phenindamine and its pharmaceutically acceptable salts are preferred exemplified materials.

The phenindamine is used as its pharmaceutically acceptable salt. Such salts may be selected from a wide range of materials and include salts from the group consisting of citrate, hydrobromide, hydrochloride, maleate, succinate, sulfate, tartrate, and mixtures thereof. Phenindamine tartrate is the preferred active form of the drug. The amount of phenindamine used in the total formulation will depend on the dosage desired which may range from 12.5 to 50 mg. This amount may be provided by employing from about 4 to 40% by weight phenindamine in the final wax matrix.

0 295 212

As discussed above, the delivery rate is effected by the quantity of the wax coating applied to the active compound coupled with the leachable component. It has been found necessary to employ at least one part wax for each part of active drug used to obtain sufficient stability of the drug, that is to avoid isomerization or oxidization to inactive species. In addition it is also useful to not employ amounts above about 3 parts wax for each part active drug since such high amounts of wax result in delayed release of the drug. The non-toxic wax must be in powdered form and be composed of materials having a melting point above about 35°C and preferably from 50 to 90°C. Waxes having lower melting points hinder subsequent granulation/milling and/or tabletting procedures by causing sticking to the punch surfaces. The waxes also have a preferred particle size wherein at least 75% are able to pass through a 100 mesh screen (U.S. standard sieve). Such sizes assure a uniformly dispersed mixture of components. Exemplary wax material include hydrogenated vegetable oil, beeswax, carnauba wax, paraffin, candelilla, ozokerite and mixtures thereof. Preferred waxes include carnauba wax and candelilla wax which have melting points of 82 to 85.5°C and 68° to 70°C respectively. When these waxes are blended with the active drug and leachable material the blend is preferably heated to from about 65° to about 75°C for the carnauba wax blend and 55° to 62°C for the candelilla wax blend. Such temperatures are most preferred ranges and fall within the broad range of about 5° to about 25°C below the melting point of the waxes used when blended with the other components.

It is also possible to employ other higher melting point waxes along with the lower melting point waxes described provided the final blend has a melting point within the desired range.

The wax is preferably employed in the final matrix in an amount of about 36 to 60% by weight of the total matrix. It should be recognized that the wax functions by protecting the active drug from being contacted with its isomerization/oxidizing agents present in the formulation, such as other drugs in the formulation or processing aids such as glidants and excipients.

Besides this function, the wax should be able to provide pathways for drug release. When high amounts of wax are employed these pathways are curtailed and fail to result in proper drug release.

The leachable component used in the matrix must be inert to the active drug, that is does not promote its isomerization or oxidation to inactive species. In addition the material must be either water swellable or water soluble so that when present in the gastrointestinal tract it disturbs the wax matrix so the drug is leached from the matrix. This disruption may occur by solubilization creating additional passages for the drug to be quickly released and/or by swelling causing disintegration of the wax matrix. Without being limited thereto exemplary materials include calcium sulfate, calcium carbonate, calcium phosphate (dibasic), sugar, starch and mixtures thereof. Materials that cannot be used include talc, hydroxypropylcellulose, modified starches, cellulose and microcrystalline cellulose. When employed in the matrix the leachable component is used in amounts of about 10 to about 60% by weight of the total matrix.

Once the final wax matrix is prepared it may be processed into final product alone or mixed with other pharmaceutical compositions and processed into final products. A preferred procedure is to mix the matrix with active ingredients such as analgesic, decongestant, and antitussive materials. Some of these materials are known to act as oxidizing agents when used with the active drug not entrapped in the wax matrix. Non-limiting illustrative examples include analgesic materials selected from the group consisting of acetaminophen, aspirin, salicylamide, phenacetin, ibuprofin and mixtures thereof, decongestant materials selected from phenylephrine hydrochloride, phenylpropanolamine, pseudoephedrine hydrochloride, ephedrine sulfate and mixtures thereof, and antitussive materials selected from dextromethorphan, dextromethorphan hydrobromide, noscapine, carbetapentane citrate, chlophedianol hydrochloride and mixtures thereof.

The formulations once prepared may be put into capsules, compressed into tablets, stored for future use or formulated with conventional carriers, to prepare pharmaceutical compositions which offer a variety of administration and dosages suitable for particular applications. Such compositions may be in the form of a lozenge, tablet, toffee, nougat, chewy candy, chewing gum, and so forth. The carriers may be selected from a wide range of materials. Without being limited thereto, such materials include bulking agents such as fillers, diluents, binders and adhesives, lubricants, disintegrants, colorants, flavorings, sweeteners and miscellaneous materials such as buffers and adsorbents in order to prepare a particular pharmaceutical composition. The preparation of confectionery and chewing gum products is historically well known and has changed very little over the years.

Lozenges are flavored medicated dosage forms intended to be sucked and held in the mouth. They may be in the form of various shapes, the most common being flat, circular, octagonal and biconvex forms. The lozenge bases are generally in two forms, hard, boiled candy lozenges and compressed tablet lozenges.

The hard boiled candy lozenges are prepared from a mixture of sugar and other carbohydrates that are kept in an amorphous or glassy condition. This form can be considered a solid syrup of sugars generally having from 0.5 to 1.5% moisture. Such materials normally contain up to 92% corn syrup, up to 55% sugar and from 0.1% to 5.0% water. The syrup component generally is prepared from corn syrups high in fructose, but may include other materials. Further ingredients such as flavorings, sweeteners, acidulents, colorants and so forth may also be added.

Boiled candy lozenges may also be prepared from non-fermentable sugars such as sorbitol, mannitol, and hydrogenated corn syrup. A typical hydrogenated corn syrup is Lycasin (trademark of Roquette Feres). The candy lozenges may contain up to about 95% sorbitol, a mixture of sorbitol and mannitol at a ratio of about 9.5 to 0.5 up to about 7.5 to 2.5 and hydrogenated corn syrup up to about 55% of the syrup component.

In contrast, compressed tablet lozenges contain particular materials and are formed into structures under

4

pressure. They generally contain sugars in amounts up to 95% and typical tablet excipients such as binders and lubricants as well as flavors, colorants and so forth.

The lozenges may be made of soft confectionary materials such as those contained in nougat. These materials contain two primary components, namely a high boiling syrup such as corn syrup or the like, and a relatively light textured frappe, generally prepared from gelatin, egg albumen, milk proteins such as casein, and vegetable proteins such as soy protein, and the like. The frappe is generally relatively light, and may, for example, range in density from about 0.5 to about 0.7g/cc.

By comparison, the high boiling syrup, or "bob syrup," is relatively viscous and possesses a higher density, and frequently contains a substantial amount of sugar. Conventionally, the final nougat composition is prepared by the addition of the "bob syrup" to the frappe under agitation, to form the basic nougat mixture. Further ingredients such as flavorings, oils, additional sugar and the like may be added thereafter also under agitation. A general discussion of the composition and preparation of nougat confections may be found in B.W. Minifie, CHOCOLATE, COCOA AND CONFECTIONERY: Science and Technology, 2nd edition, AVI Publishing Co., Inc., Westport, Connecticut, (1980), at Pages 424-425.

Tablets of this invention may also be in chewable form. This form is particularly advantageous because of convenience and patient acceptance and rapid onset of bioactivity. To achieve acceptable stability and quality as well as good taste and mouth feel several considerations are important, namely amount of active substance per tablet,. flavor, compressibility and organoleptic properties of the drug.

The preparation of chewable medicated candy is prepared by procedures similar to those used to make soft confectionery. This procedure generally involves the formation of a boiled sugar-corn syrup blend to which is added a frappe mixture. The boiled sugar-corn syrup blend may be prepared from sugar and corn syrup blended in parts by weight ratio of 90 to 10 : 10 to 90. This blend is heated to temperatures above 250°F to remove water and to form a molten mass. The frappe is generally prepared from gelatin, egg albumen, milk proteins such as casein, and vegetable proteins such as soy protein, and the like which are added to a gelatin solution and rapidly mixed at ambient temperature to form an aerated sponge like mass. The frappe is then added to the molten candy base and mixed until homogenous at temperatures between 150°F and 250°F. The leachable wax matrix can then be added as the temperature of the mix is lowered to around 120°F to 194°F whereupon additional ingredients are added such as flavors, and colorants. The formulation is further cooled and formed to pieces of desired dimensions.

A general discussion of the lozenge and chewable tablet forms of confectionery may be found in H.A. Lieberman and L. Lachman, Pharmaceutical Dosage Forms: Tablets Volume 1, Marcel Dekker, Inc., New York, N.Y. at pages 289 to 466, incorporated herein by reference.

With regard to the chewing gum formulation in particular, the amount of gum base employed will vary greatly depending on various factors such as the type of base used, consistency desired and other components used to make the final product. In general, amounts of about 5% to about 45% by weight of the final chewing gum composition are acceptable for use in chewing gum compositions with preferred amounts of about 15% to about 25% by weight. The gum base may be any water insoluble gum base well known in the art. Illustrative examples of suitable polymers in gum bases include both natural and synthetic elastomers and rubbers. For example, those polymers which are suitable in gum bases, include, without limitation, substances of vegetable origin such as chicle, jelutong, gutta percha and crown gum. Synthetic elastomers such as butadiene-styrene copolymers, isobutylene-isoprene copolymers, polyethylene, polyisobutylene and polyvinylacetate and mixtures thereof, are particularly useful.

The gum base composition may contain elastomer solvents to aid in softening the rubber component. Such elastomer solvents may comprise methyl, glycerol or pentaerythritol esters of rosins or modified rosins, such as hydrogenated, dimerized or polymerized rosins or mixtures thereof. Examples of elastomer solvents suitable for use herein include the pentaerythritol ester of partially hyrogenated wood rosin, pentaerythritol ester of wood rosin, glycerol ester of wood rosin, glycerol ester of partially dimerized rosin, glycerol ester of polymerized rosin, glycerol ester of tall oil rosin, glycerol ester of wood rosin and partially hydrogenated wood rosin and partially hydrogenated methyl ester of rosin, such as polymers of alpha-pinene or beta-pinene; terpene resins including polyterpene and mixtures thereof. The solvent may be employed in an amount ranging from about 10% to about 75% and preferable about 45% to about 70% by weight to the gum base.

A variety of traditional ingredients such as plasticizers or softeners such as lanolin, stearic acid, sodium stearate, potassium stearate, glyceryl triacetate, glycerine and the like for example, natural waxes, petroleum waxes, such as polyurethene waxes, paraffin waxes and microcrystalline waxes may also be incorporated into the gum base to obtain a variety of desirable textures and consistency properties. These individual additional materials are generally employed in amounts up to about 30% by weight and preferably in amounts from about 3% to about 20% by weight of the final gum base composition.

The chewing gum composition may additionally include the conventional additives of flavoring agents, coloring agents such as titanium dioxide; emulsifiers such as lecithin and glyceryl monostearate; and additional fillers such as aluminum hydroxide, alumina, aluminum silicates, calcium carbonate, and talc and combinations thereof. These fillers may also be used in the gum base in various amounts. Preferably the amount of fillers when used will vary from about 4% to about 30% by weight of the final chewing gum.

In the instance where sweeteners are utilized, the present invention contemplates the inclusion of those sweeteners well known in the art, including both natural and artificial sweeteners. Thus, sweeteners may be chosen from the following non-limiting list;

A. Water-soluble sweetening agents such as monosaccharides, disaccharides and polysaccharides such as xylose, ribose, glucose, mannose, galactose, fructose, dextrose, sucrose, sugar, maltose, partially hydrolyzed starch, or corn syrup solids and sugar alcohols such as sorbitol, xylitol, mannitol and mixtures thereof.

B. Water-soluble artificial sweeteners such as the soluble saccharin salts, i.e., sodium, or calcium saccharin salts, cyclamate salts, sucralose acesulfame-K and the like, and the free acid form of saccharin.

C. Dipeptide based sweeteners such as L-aspartylphenylalanine methyl ester and materials described in U.S. Patent No. 3,492,131 and the like.

In general, the amount of sweetener will vary with the desired amount of sweeteners selected for a particular chewing gum. This amount will normally be about 0.001% to about 90% by weight when using an easily extractable sweetener. The water-soluble sweeteners described in category A above, are preferably used in amounts of about 25% to about 75% by weight, and most preferably from about 50% to about 65% by weight of the final chewing gum composition. In contrast, the artificial sweeteners described in categories B and C are used in amounts of about 0.001% to about 5.0% and most preferably about 0.05 to about 2.5% by weight of the final chewing gum composition. These amounts are ordinarily necessary to achieve a desired level of sweetness independent from the flavor level achieved from flavor oils. While water may be added independently with dry sweeteners, it will generally be added as part of a corn syrup or corn syrup mixture.

Suitable flavorings include both natural and artificial flavors, and mints such as peppermint, menthol, artificial vanilla, cinnamon, various fruit flavors, both individual and mixed, and the like are contemplated. The flavorings are generally utilized in amounts that will vary depending upon the individual flavor, and may, for example, range in amounts of about 0.5% to about 3% by weight of the final composition weight.

The colorants useful in the present invention, include the pigments such as titanium dioxide, that may be incorporated in amounts of up to about 1% by weight, and preferably up to about 0.6% by weight. Also, the colorants may include other dies suitable for food, drug and cosmetic applications, and known as F.D. & C. dyes and the like. The materials acceptable for the foregoing spectrum of use are preferably water-soluble. Illustrative examples include indigoid die, known as F.D. & C. Blue No. 2, which is the disodium salt of 5,5'-indigotindisulfonic acid. Similarly, the dye known as F.D. & C. Green No. 1, comprises a triphenylmethane dye and is the monosodium salt of 4-[4-Nethyl-p-sulfobenzylamino)diphenylmethylene]-[1-(N-ethyl-N-p-sulfoniumbenzyl)-2,5-cyclohexadienimine]. A full recitation of all F.D. & C. and D. % C. and their corresponding chemical structures may be found in the Kirk-Othmer Encyclopedia of Chemical Technology, 3rd Edition in Volume 6, at Pages 561-595, which text is accordingly incorporated herein by reference.

Suitable oils and fats that are useable would include partially hydrogenated vegetable or animal fats, such as coconut oil, palm kernel oil, beef tallow, lard, and the like. These ingredients are generally utilized in amounts with respect to the comestible product of up to about 7.0% by weight, and preferably up to about 3.5% by weight of the final product.

The present invention is further illustrated by the following examples. All parts and percentages in the examples and throughout the specification and claims are by weight unless otherwise indicated.

## Example I

This Example demonstrates the formation of the wax melt containing phenindamine by the inventive process.

A dry blend was prepared containing powdered carnauba wax (40 w/w%), phenindamine tartrate powder (25 w/w%) and powdered anhydrous calcium sulfate (35 w/w%). The dry mixed powders were then put into a steam jacketed mixer for 5 minutes at speed 40-45 rpm. Then with mixing, the bowl is heated to 65-75°C for 10-12 minutes and mixing continued for an additional 1-2 minutes until a slightly soft granular consistency is obtained. The wax melt granulation (35-55°C) was discharged and spread on paper lined trays to cool. After cooling to room temperature (25°C) the granular product was milled to the desired particle size and stored for future use.

The percent phenindamine released from the wax blend was determined by placing 100 milligrams of powder blend into 80 milliliters of DI water at 37°C and rotated at 40 rpm for 45 minutes. The phenindamine content in solution was then analyzed. The results for three granular products having different particle size distributions are set forth in Table 1. Group A released 87.93% phenindamine; Group B released 78.1% phenindamine; and Group C released 69.6% phenindamine.

## TABLE 1

Group

| | | 20 | 40 | 60 | 80 | 100 | PAN |
|---|---|---|---|---|---|---|---|
| A | % retained on<br>U.S. std. sieve | 0.00 | 2.29 | 25.43 | 18.11 | 38.57 | 15.60% |
| B | % retained on<br>U.S. std. sieve | 1.14 | 31.14 | 28.57 | 10.80 | 5.20 | 23.15% |
| C | % retained on<br>U.S. std. sieve | 14.94 | 34.34 | 21.14 | 8.69 | 4.43 | 16.46% |

### Example II

This example demonstrates the formation of tablets using the leachable wax matrix of this invention.

The product of Example I (Group A) blended with acetaminophen and pseudoephedrine. Tablets were compressed at 2 tons pressure, maximum. The tablets contained 9.06% phenindamine, 5.44% pseudoephedrine and 65.43% acetaminophen plus other tabletting excipients.

Tablets were stored at 25°C and 80% relative humidity (RH) for one week without closure and the phenindamine content determined by USP Dissolution. Separately tablets were also stored in the open at 37°C and 80% RH for one week and tested for USP Dissolution. The tablets exhibited good stability and virtually no isomerization at these conditions. The tablets exhibited a 91.84% and 82.8% dissolution, respectively, when performed according to USP Dissolution Apparatus Method II at 37°C, 75 RPM for 45 minutes in water.

The invention being thus described, it will be obvious that the same may be varied in many ways. Such variations are not to be regarded as a departure from the spirit and scope of the invention and all such modifications are intended to be included within the scope of the following claims.

## Claims

1. A process for preparing a leachable wax matrix granulation containing an active pharmaceutical composition, which comprises:
   a) blending the active pharmaceutical composition with a powdered non-toxic wax and a leachable filler until a uniform mixture is obtained,
   b) heating the mixture to a temperature below the melting point of the non-toxic wax until the wax becomes softened,
   c) mixing the mixture to form a uniform homogenous granulation and
   d) recovering the free-flowing granulation.

2. The process of claim 1 wherein the non-toxic wax has at least 75% particles which pass through a 100 mesh U.S. standard sieve.

3. The process of the claims 1 to 2 wherein heating step (b) and mixing step (c) are performed simultaneously.

4. The process according to anyone of the claims 1 to 3 wherein the active pharmaceutical composition is a material which is unstable in the presence of water or air.

5. The proces according to anyone of the claims 1 to 4 wherein the leachable filler is selected from the group consisting of calcium sulfate, calcium carbonate, calcium phosphate, sugar, starch and mixtures thereof.

6. The process according to anyone of the claims 1 to 5 wherein the active pharmaceutical composition is phenindamine, procaterol and their pharmaceutically acceptable salts.

7. The process in claim 6 wherein the pharmaceutically acceptable salts of phenindamine are selected from the group consisting of citrate, hydrobromide, hydrochloride, maleate, succinate, sulfate, tartrate, and mixtures thereof.

8. The process according to anyone of the claims 1 to 7 wherein the non-toxic wax is selected from the group consisting of hydrogenated vegetable oil, beeswax, carnauba wax, paraffin, candelilla, ozokerite, and mixtures thereof.

9. The process according to anyone of the claims 1 to 8 wherein the active pharmaceutically composition is present in the wax matrix in an amount of at least 1 part wax to 1 part active.

10. The process according to anyone of the claims 1 to 9 wherein the free-flowing granulation contains about 36 to 60 % wax, about 4 to about 40 % active pharmaceutical composition, and about 10 to about 60 % filler as the leachable component.

11. The process of claim 8 wherein the non-toxic wax has a melting point above about 35°C.

12. A free-flowing granulation containing an active pharmaceutical composition produced by the process according to anyone of the claims 1 to 11.

13. Unit dosage forms made from or containing a granulation as obtained by anyone of the claims 1 to 11.

14. A unit dosage in the form of a lozenge, tablet, confectionary, toffee, nougat, chewy candy of chewing gum as claimed in claim 13.

15. A unit dosage form according to the claims 13 or 14 wherein at least one carrier is used selected from fillers, diluents, binders and adhesives, lubricants, disintegrants, colorants, flavorings, sweeteners, buffers and adsorbents.